# EUROPEAN PATENT SPECIFICATION

(11) **EP 0 411 063 B1**
(45) Date of publication and mention of the grant of the patent: **23.10.1996**
(21) Application number: 89910504.3
(22) Date of filing: 14.09.1989
(51) Int. Cl.: A61F 13/02

(54) **WOUND DRESSING**
WUNDVERBAND
PANSEMENT

(30) Priority: 26.09.1988 US 248677
(43) Date of publication of application: 06.02.1991
(73) Proprietor: Paul Hartmann Aktiengesellschaft, D-89522 Heidenheim (DE)
(72) Inventor: CARTMELL, James, V., Centerville, OH 45458 (US); WOLF, Michael, L., West Milton, OH 45383 (US); STURTEVANT, Wayne, R., Centerville, OH 45458 (US); VALADEZ, Manuel, J., Castaic, CA 91384 (US)
(74) Representative: Becker, Maria, Dipl.-Phys.
(86) International application number: US8903913
(87) International publication number: WO9003155

(56) References cited:
- EP-A- 0 171 800
- EP-A- 0 236 104
- EP-A- 0 249 694
- GB-A- 1 510 569
- US-A- 4 226 232
- US-A- 4 285 338
- US-A- 4 399 816

## Description

The invention herein is directed to a wound dressing and, more particularly, to a wet, clear, flexible wound dressing. The wound dressing can be contoured to a wound and will provide absorbency while maintaining the wound wet. The wound dressing herein is easily removable from the wound without deleteriously affecting the healing of the wound.

Managing draining wounds such as decubitus ulcers has presented a difficult problem of treatment for the medical profession. It has been difficult to maintain the wounds free of the secretion to permit them to heal. The accumulation of exudate, such as blood, serum and purulent matter in the crevices of a wound can lead to bacterial growth which can delay healing of the wound.

Currently there are wound treatment compositions which are comprised of hydrogel materials in powder form. Generally, such dry, powdery hydrogel materials are introduced to an open, draining wound to absorb the exudate from the wound. One such commercially available method of treatment employing dry hydrogel material is the use of Dextranomer beads. Dextranomer beads are highly hydrophyllic and comprise spherical beads which can be introduced to a wound site to absorb the wound exudate. A drawback of such hydrogel material is that the dry material can tend to clump and form lumps prior to and during introduction of the material to the wound site. The clumping or lumping can also occur after introduction of the material to the wound site and as it absorbs the wound exudate. The lumps or granules are difficult to apply evenly to the wound and, subsequently, are difficult to remove from the wound site without damaging the new tissue that forms at the wound site.

U.S.-A-4,266,232 teaches the blending of a hydrogel material with a liquid curing agent, such as a polyethylene glycol prior to introducing the gel-like or salve-like material to a wound. Again, there are drawbacks with such a system, as the system cannot be sterilized by irradiation due to the formation of free radicals within the gel material.

A wound dressing is described in EP-A-0 236 104 which comprises a waterproof, water-vapour-permeable polymer film secured to a polymer foam frame by a pressure sensitive adhesive coated on one side of the frame and extending outwards beyond the film to provide for attaching the dressing to the skin about a wound. An absorbent material may also be secured within the foam frame and over the film, by means of the adhesive, to form a wound facing layer. The wound facing side of the dressing may be protected by a release liner attached to the adhesive.

A need has arisen for a wound dressing which can provide a protective covering to the wound while being able to absorb the exudate from the wound. It would be desirable to have a wound dressing which could provide a protective pad over the wound to prevent debris and foreign matter from contaminating the wound and which could cushion the wound against pressure. It would be desirable to have a wound dressing which would not adhere to the new tissue forming in the wound or the exudate being released by the wound. It would be desirable to have a wound dressing which would be clear to enable observation of the healing process of the wound, but which would shield the wound against bacteria to inhibit infection. It would also be desirable to have a wound dressing which, by selecting a suitable carrier film, could either permit moisture transfer so that the wound environment is stabilized with respect to moisture presence or occlude moisture transfer.

It would also be desirable to provide a wound dressing which could be precut, sterilized and readily available for application to a draining wound. Such a wound dressing could be readily applied by an attendant without the need for mixing and applying a paste or gel to an open wound. Such a wound dressing system could save time and expense and insure a uniform, consistent coating. It would further be desirable to have such a wound dressing which could be radiation sterilized as current gas sterilization techniques are coming under more and more restrictions and closer scrutiny for environmental reasons.

From one aspect, the present invention consists in a wound dressing comprising a flexible backing layer having a pressure-sensitive adhesive layer, an absorbent material on the wound side of the backing layer, and a release liner extending over the exposed pressure-sensitive adhesive layer and the absorbent material, which release liner has a selective releasability whereby it can be removed from the wound dressing intact to leave portions of the pressure-sensitive adhesive and the absorbent material exposed, characterised by a depression on the wound side of the dressing and hydrogel material constituting the absorbent material contained in the depression.

The invention provides a wound dressing which can be manufactured to any desirable size to produce a dressing for any size open, draining wound. It enables the provision of a wound dressing which will absorb the exudate from the wound but which will not adhere to the wound. Thus, when it is removed from the wound it will not damage the wound. The wound dressing may be a clear, wet wound dressing which allows visual inspection of the wound without having to remove the dressing.

The backing layer may be vacuum formed to produce the depression on its wound side. Alternatively, the depression may be formed by a perimeter-defining, damming layer which overlies the backing layer.

The hydrogel material may include from about 20% to about 35% by weight of a plasticizer selected from a group consisting of polypropylene glycol, polyethylene glycol and glycerine, from about 8% to about 12% by weight isophoronediisocyanate terminated prepolymer with about 3% NCO content, from about 5% to about 7% by weight polyethylene dioxide based diamine, up to about 1% by weight of a salt and the remaining percentage being water.

The release liner extends over the exposed pressure sensitive adhesive layer and the exposed hydrogel. It has a selective releasability such that it can be removed from the wound dressing to expose the pressure sensitive adhesive and the hydrogel. The pressure sensitive adhesive is exposed along an area which forms a perimeter surrounding the hydrogel.

From another aspect, the invention consists in a method of forming a wound dressing having a flexible film coated with a pressure-sensitive adhesive capable of adhering to the skin of a patient, an absorbent material and a release liner extending over the pressure sensitive adhesive and absorbent material, characterised by the steps of introducing the non-adhesive coated side of the adhesive coated flexible film to a vacuum platen having a cavity formed therein, pulling a vacuum on the vacuum platen to draw the adhesive coated flexible film into the cavity, dispensing an absorbent material in the form of a hydrogel into the cavity formed in the adhesive coated flexible film, and attaching the release liner over the adhesive coated flexible film, hydrogel exposed surface.

The invention enables the manufacture of a wound dressing which is wet, clear and radiation sterilizable. The flexible film can be occlusive or permeable to moisture vapor flow. The hydrogel can be in a fluid state when it is introduced to the cavity. It cures or sets to a gel-like consistency, after which the vacuum is withdrawn. The depression remains containing the gel-like hydrogel material. The release liner can be applied to cover the exposed adhesive surface of the flexible film and the exposed hydrogel in the cavity. It can have a selective releasability such that it can be removed from the adhesive and hydrogel without tearing or violating the integrity of the hydrogel or adversely impacting the adhesive properties of the pressure sensitive adhesive.

Embodiments of the invention will now be described, by way of example, with reference to the accompanying drawings, in which:-
Figure 1 is a plan view of the wound dressing viewing the surface which is to be applied to the patient;
Figure 2 is a cross-sectional view of the wound dressing of Figure 1 taken along line 2-2;
Figure 3 is a plan view of another embodiment of a wound dressing as viewed from the exposed side of the wound dressing when the wound dressing is applied to a patient;
Figure 4 is a plan view of another embodiment of the wound dressing showing the non-patient side of the dressing;
Figure 5 is a cross-sectional view of the wound dressing embodiment shown in Figure 4 taken along line 5-5;
Figure 6 is a perspective view of a vacuum platen which can be used to form the wound dressing such as shown in Figure 1; and
Figure 7 is a perspective view illustrating the method of forming a wound dressing such as shown in Figure 1.

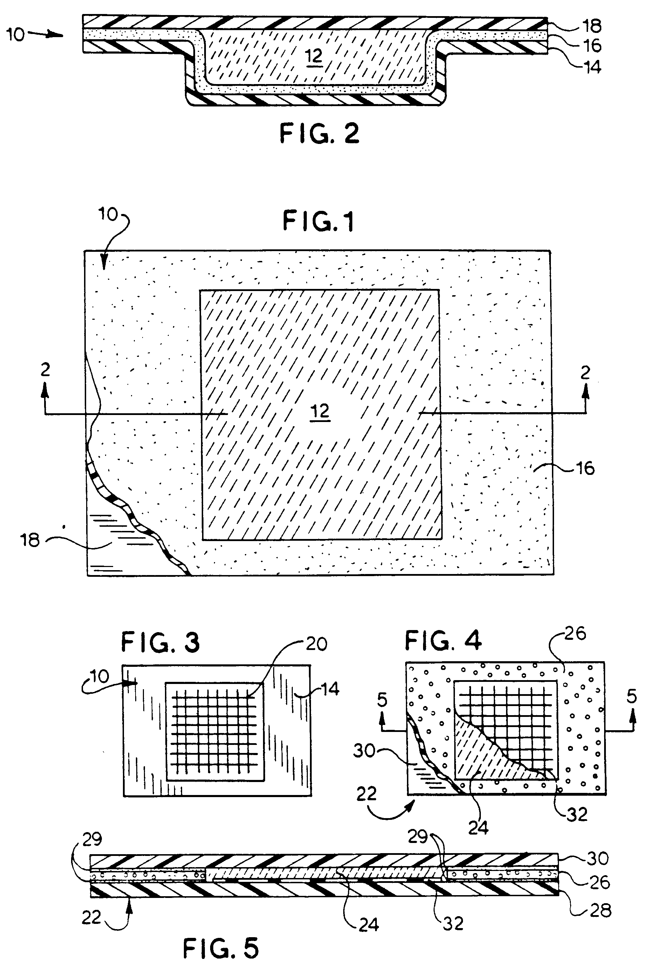

The wound dressing herein will be described with regard to the accompanying drawings. In particular, with reference to Figures 1 through 3, preferred embodiments of the wound dressing 10 is illustrated. The wound dressing uses a hydrogel 12 which will be in contact with the wound when the wound dressing is placed on a patient. The hydrogel is maintained in a cavity which is formed in a flexible membrane 14. The flexible membrane 14 serves as the carrier or substrate for the hydrogel. The flexible membrane also serves as a protective layer for the wound dressing when the wound dressing is applied to a wound on a patient. The flexible membrane can be selected from a material which is moisture vapor permeable so that when the wound dressing is placed on a wound, it will permit the hydrogel and thereby the wound covered by the hydrogel to release moisture. The use of a moisture vapor permeable material prevents undue moisture build-up at the wound site. In some instances it is desirable to use an occlusive film to retain moisture in the healing wound.

The flexible membrane 14 also serves as a substrate which can aid in the adhering of the wound dressing to the patient. The flexible membrane is coated with an adhesive layer 16. The adhesive layer 16 can be any suitable adhesive and preferably a pressure-sensitive adhesive that is capable of being in contact with the human body without causing any harmful affects. Acceptable pressure-sensitive adhesives include acrylate based adhesives.

The hydrogel is positioned within a cavity of the flexible membrane. The cavity is formed within the perimeter of the side edges of the flexible membrane, creating a perimeter of exposed flexible membrane around the hydrogel. The exposed flexible membrane provides an exposed surface area surrounding the hydrogel which exposed surface area is coated with the adhesive. The adhesive layer forming a perimeter around the hydrogel aids in the securing of the wound dressing to a patient. The exposed hydrogel also serves as an anchoring adhesive for the wound dressing on the patient. Thus, the hydrogel and pressure-sensitive adhesive provide two distinct anchoring adhesives for the wound dressing.

Referring to Figure 2, a protective release liner 18 can be laminated onto the wound dressing to protect the pressure-sensitive adhesive and the hydrogel of the wound dressing prior to the wound dressing being applied. The protective release liner is a removable protective release liner which has a selective releasability such that it can be readily removed from its contact with the pressure sensitive adhesive and the hydrogel without destroying the adhesive properties of the pressure-sensitive adhesive or destroying the integrity of the hydrogel.

The flexible membrane 14 can be constructed from any suitable material which can provide a backing to a wound dressing. The flexible membrane can be a polymeric elastic or flexible film coating providing a bacterial barrier and formed from a water vapor permeable pliable elastomer material such as a flexible polyurethane, polyacrylate, polyethylene and the like. A polyurethane film is the preferred material for the flexible membrane 14. For an occlusive film a polypropylene or co-polyester can be used.

The hydrogel is a hydrogel material which comprises from about 20 to about 35% by weight of a polyhydric alcohol selected from the group consisting of polypropylene glycol, polyethylene glycol and glycerine. The hydrogel further includes from about 8 to about 12% of an isophoronediisocyanate terminated prepolymer with about 3% NCO content. The hydrogel also includes from about 5 to about 7% by weight of a diamine, with the preferred diamine being a polyethylene oxide based diamine. The hydrogel also includes up to about 1% by weight of a salt such as sodium chloride. The balance of the hydrogel material is comprised of water.

The manufacture of similar hydrogel material is disclosed in U. S. Patent No. 4,517,326, the disclosure of which is incorporated herein by this reference. A similar method can be used to create the hydrogel herein except for the material contents.

A particularly preferred hydrogel composition is from about 25 to 30% by weight glycerine, from about 9.5 to about 10.5% by weight of an isophoronediisocyanate terminated prepolymer with about 3% NCO content, about 6% by weight polyethylene oxide based diamine, 0.9% by weight sodium chloride, with the balance being water.

The hydrogel composition herein is particularly suited for being a wound dressing. The hydrogel is a wet hydrogel due to it containing more than 50% by weight water. The hydrogel is capable of providing some adhesiveness to the wound dressing. However, the adhesive property of the hydrogel is not such that it will damage cell or tissue growth deleteriously, upon removal of the dressing. That is, the hydrogel provides an adhesive tenacity to aid in adhering the wound dressing to the patient and wound site. The hydrogel exhibits a high degree of fluid absorption and can thereby absorb a sufficiently large quantity of wound exudate.

The hydrogel composition herein retains its gel-like integrity upon removing the wound dressing from a wound site. The hydrogel does not leave debris in the wound upon removal such as hydrogel particles. The hydrogel composition herein also exhibits a capability of non-traumatically releasing from the wound when the wound dressing is removed from the wound. This non-traumatic release of the hydrogel wound dressing from the wound does not destroy the new cell tissue forming at the wound site and thereby wound healing is not inhibited when the dressing is removed. The hydrogel material can also provide a protective cushioning of the wound due to its gel-like consistency.

Another advantage of the hydrogel herein is its ability to absorb water. It can remain on a wound for relatively long periods of time and, therefore, does not need to be removed frequently.

A special advantage of the hydrogel material herein is that the hydrogel material is clear. That is, the hydrogel material is not only translucent but also transparent. The hydrogel material is sufficiently clear such that visual inspection of the wound can be performed without having to remove the wound dressing. Although the hydrogel material does not deleteriously affect the wound when it is removed, it is still highly desirable to avoid removing dressings from a wound site, as removal can provide an opportunity for the ingress of bacteria to the wound from the surrounding environment.

Another particular benefit of providing a clear hydrogel as a wound dressing is that a wound sizer can be incorporated in the wound dressing. With regard to Figure 3, there is shown another embodiment of a wound dressing herein. The wound dressing 10 shown in Figure 3 uses similar reference numerals to refer to the similar components as discussed with regard to the wound dressing embodiment shown in Figures 1 and 2. Figure 3 shows a plan view of a wound dressing 10 looking at the non-patient, contact surface of the wound dressing. The wound dressing 10 has a flexible membrane 14 and an rectangular hydrogel area. Printed on the flexible membrane 14 is a grid which functions as a wound sizer 20. The wound sizer 20 can have any grid-like pattern which can be used for measuring the size of a wound. Shown in Figure 3 is a rectangular grid pattern, but a circular grid pattern could also be used. The clear hydrogel permits observation of the wound, and the wound sizer printed on the flexible membrane permits observation of changes in the wound size while the wound dressing is in use.

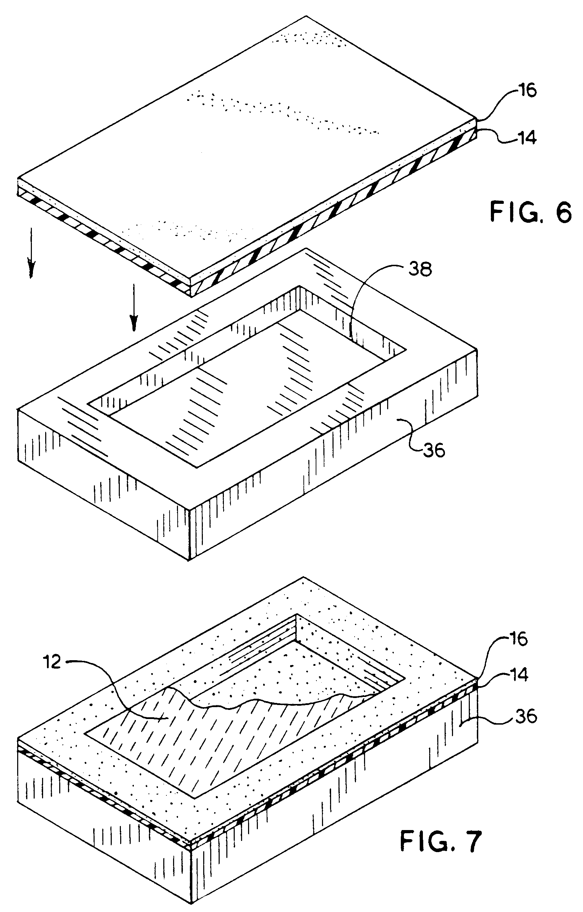

A step in the manufacturing process of the wound dressing shown in Figures 1 through 3 is illustrated in Figures 6 and 7. With regard to Figure 6, there is shown an exploded view of a processing step in the manufacturing process for the wound dressing. The wound dressing is manufactured using a vacuum platen 36 which has a cavity 38 formed thereon. The cavity 38 is cut into the platen in any size that is desirable for the hydrogel component of the wound dressing. The size of the cavity can be selected based upon the end use of the wound dressing. For the hydrogel material herein, the size can vary as the hydrogel material readily cures and maintains its integrity, regardless of the area of the hydrogel when formed to a depth sufficient for the wound dressings herein.

The flexible membrane, with the adhesive side facing upwardly, is placed in contact with the vacuum platen. A vacuum pump (not shown) in communication with the platen, creates a partial vacuum in the platen which is sufficiently strong to form the flexible membrane 14 to the contour of the cavity 38 in the vacuum platen 36. The partial vacuum is also sufficient to hold the flexible membrane in place against the vacuum platen, as the flexible membrane assumes the size and shape of the cavity.

Upon forming the cavity, the hydrogel material is dispensed into the cavity overlying and covering the adhesive coating on the flexible membrane. The hydrogel is dispensed to uniformly fill the cavity. The vacuum is maintained until the hydrogel sufficiently sets so that movement of the flexible membrane does not violate the integrity of the hydrogel, nor does the hydrogel tend to flow or run out of the cavity. Generally, the vacuum need not be maintained as the weight of the hydrogel is sufficient force to retain the shape of the cavity when using the thin films employed herein. Generally, the hydrogel is formed in about a 1/8 inch thickness which is suitable for most wounds, but other thicknesses can be used depending upon the final use of the wound dressing.

Figure 7 shows the adhesive-coated flexible membrane formed in the vacuum platen 36 with the hydrogel 12 filling the cavity and leaving an adhesive coated edge of the flexible membrane exposed around the hydrogel. The hydrogel herein readily sets such that upon release of the vacuum the hydrogel will retain its integrity such that movement of the film/hydrogel interface will not disturb the integrity of the hydrogel layer which remains substantially intact. A protective cover or release line 18 can be placed over the assembly and the entire construction can be die cut to the desired overall size for the wound dressing.

Another embodiment of the wound dressing herein is shown in Figures 4 and 5. Figure 4 shows a wound dressing 22 which includes a hydrogel layer 24 forming a wound covering and wound exudate absorbing layer. The hydrogel layer 24 can be a hydrogel material such as discussed with regard to Figures 1 through 3.

The hydrogel layer 24 is formed over a carrier or substrate layer 28 which can be constructed of any moisture vapor permeable material such as a polyurethane. The substrate layer 28 is similar to the flexible membrane 14 in the embodiment shown in Figures 1 through 3 and can be any of the materials described with regard to that embodiment. The structure for the wound dressing shown in Figure 4 is shown in the cross-sectional view of Figure 5.

With regard to Figures 4 and 5, the hydrogel layer 24 is maintained in place on the substrate layer 28 by a dam, such as a foam dam 26 which overlies the substrate layer 28. The foam dam 26 has a sufficient height to support the hydrogel layer when the hydrogel material is deposited on the substrate layer 28. The foam dam 26 can be constructed of any suitable material which will be biocompatible with the body. A preferred material is polyethylene foam. The foam dam can be coated on both of its surfaces, with a suitable adhesive 29. The adhesive coated on the patient side can be different than the adhesive on the substrate layer side. That is, the adhesive properties for adhering the foam dam to the substrate layer may be different than those for adhering the foam dam to a patients skin. A release liner 30 can be coated over the exposed hydrogel and foam dam member.

For adhering the wound dressing to a patient, the foam dam member can be coated with a pressure-sensitive adhesive on its surface facing the release liner. The pressure-sensitive adhesive can be a pressure-sensitive adhesive such as described with regard to the embodiment in Figures 1 and 2. The hydrogel material also can act as an adhesive to aid in adhering the wound dressing to a patient.

The release liner 30 can be any suitable material having release properties for selectively being releasable from the hydrogel and foam dam without destroying the integrity of the hydrogel or foam dam. As shown in Figure 4, the flexible backing can be imprinted with a printed wound sizer 32. As with the earlier embodiment, the hydrogel material in the embodiment shown in Figures 4 and 5 is a clear hydrogel material which will permit viewing of the wound underneath the hydrogel material when the wound dressing is in place. The grid or printed wound sizes 32 can permit observation of the wound and monitoring of changes in size of the wound.

The hydrogel wound dressing herein provides a benefit not currently realizable in state-of-the-art wound dressings. The hydrogel material is capable of absorbing wound exudate. The hydrogel material is clear and can permit visual observation of the wound. The hydrogel herein retains its integrity such that upon removal of the wound dressing, no gel debris is left in the wound. The hydrogel material has physical properties which permit it to be non-traumatically removed from a wound. The hydrogel material also cushions the wound against pressure which can be exerted on the outer surface of the wound dressing when the wound dressing is worn by the patient. The hydrogel material herein is also advantageous in that it permits extended wearing of the dressing by a patient due to the water absorption that is provided by hydrogel material.

## Claims

1. A wound dressing (10,22) comprising a flexible backing layer (14,28) having a pressure-sensitive adhesive layer (16,29), an absorbent material (12,24) on the wound side of the backing layer, and a release liner (18,30) extending over the exposed pressure-sensitive adhesive layer (16,29) and the absorbent material, which release liner has a selective releasability whereby it can be removed from the wound dressing intact to leave portions of the pressure-sensitive adhesive (16,29) and the absorbent material (12,24) exposed, characterised by a depression on the wound side of the dressing and hydrogel material (12,24) constituting the absorbent material contained in the depression.

2. A wound dressing (22) as claimed in claim 1, wherein the depression is formed by a perimeter-defining, damming layer (26) which overlies the backing layer (28).

3. A wound dressing as claimed in claim 1 or 2, wherein the backing layer (14,28) comprises a polyurethane film.

4. A wound dressing as claimed in claim 1, 2 or 3, wherein the pressure-sensitive adhesive (16,29) comprises acrylic copolymer adhesive.

5. A wound dressing as claimed in claim 1, 2, 3 or 4, wherein the hydrogel material (12,24) comprises from about 20% to about 35% by weight of a polyhydric alcohol selected from the group consisting of polypropylene glycol, polyethylene glycol and glycerine, from about 8% to about 12% by weight isophoronediisocyanate terminated prepolymer with about 3% NCO content, from about 5% to about 7% by weight polyethylene oxide based diamine, up to about 1% by weight of a salt, and water.

6. A wound dressing as claimed in claim 5, wherein the polyhydric alcohol comprises glycerine.

7. A wound dressing as claimed in claim 6, wherein the glycerine is present in an amount from about 25% to 30% by weight.

8. A wound dressing as claimed in claim 5, 6 or 7, wherein the salt is sodium chloride.

9. A wound dressing as claimed in any preceding claim, wherein the hydrogel (12,24) comprises a clear hydrogel layer.

10. A wound dressing as claimed in any preceding claim, including a printed wound sizer (20,32) on the backing layer (14,28) and overlying the hydrogel material (12,24).

11. A method of forming a wound dressing (10) having a flexible film (14) coated with a pressure-sensitive adhesive (16) capable of adhering to the skin of a patient, an absorbent material (12) and a release liner (18) extending over the pressure-sensitive adhesive and absorbent material, characterised by the steps of introducing the non-adhesive coated side of the adhesive coated flexible film (14) to a vacuum platen (36) having a cavity (38) formed therein, pulling a vacuum on the vacuum platen to draw the adhesive coated flexible film (14) into the cavity, dispensing an absorbent material in the form of a hydrogel (12) into the cavity formed in the adhesive coated flexible film, and attaching the release liner (18) over the adhesive coated flexible film, hydrogel exposed surface.

12. A method as claimed in claim 11, wherein the hydrogel material (12) comprises from about 20% to about 35% by weight polyhydric alcohol selected from the group consisting of polypropylene glycol, polyethylene glycol and glycerine, from about 8% to about 12% by weight isophoronediisocyanate terminated prepolymer with about 3% NCO content, from about 5% to about 7% by weight polyethylene oxide based diamine, up to about 1% by weight of a salt, and water.

13. A method as claimed in claim 12, wherein the salt is sodium chloride.

14. A method as claimed in claim 11, 12 or 13, wherein the hydrogel (12) comprises from about 25% to 30% by weight of glycerin.

15. A method as claimed in any preceding claim 11 to 14, including the step of printing a wound sizer (20) on the flexible film (14) and overlying the hydrogel material (12).

## Patentansprüche

1. Wundabdeckung (10, 22), die eine flexible Trägerschicht (14, 28) mit einer drucksensitiven, haftenden Schicht (16, 29), einem absorbierenden Material (12, 24) auf der der Wunde zugewandten Seite der Rückenbeschichtung und einer Abziehlage (18, 30) aufweist, die sich über die ungeschützte, drucksensitive, haftende Schicht (16, 29) und das absorbierende Material erstreckt, wobei die Abziehlage eine gezielte Ablösbarkeit besitzt, wodurch es unbeschädigt von der Wundabdeckung entfernt werden kann und Bereiche des drucksensitiven Klebemittels (16, 29) und des absorbierenden Materials (12, 24) ungeschützt läßt, **gekennzeichnet** durch eine Vertiefung auf der der Wunde zugewandten Seite der Abdeckung und ein Hydrogelmaterial (12, 24), welches das in der Vertiefung enthaltene absorbierende Material bildet.

2. Wundabdeckung (22) nach Anspruch 1, bei der die Vertiefung durch eine einen äußeren Umfang definierende, dämmende Schicht (26) gebildet wird, die die Trägerschicht (28) bedeckt.

3. Wundabdeckung nach einem der Ansprüche 1 oder 2, bei der die Trägerschicht (14, 28) einen Polyurethanfilm aufweist.

4. Wundabdeckung nach einem der Ansprüche 1, 2 oder 3, bei der das drucksensitive Klebemittel (16, 29) acrylisches Copolymer als Klebemittel aufweist.

5. Wundabdeckung nach einem der Ansprüche 1, 2, 3 oder 4, bei der das Hydrogelmaterial (12, 24) etwa 20 bis 35 Gew.-% eines Polyalkohols, ausgewählt aus der Gruppe, die Polypropylenglycol, Polyethylenglycol und Glycerin umfaßt, etwa 8 bis 12 Gew.-% durch Isophorondiisocyanat terminiertes Vorpolymerisat mit einem Gehalt von etwa 3 % NCO, etwa 5 bis 7 Gew.-% auf Polyethylenoxid basierendes Diamin, bis etwa 1 Gew.-% Salz und Wasser aufweist.

6. Wundabdeckung nach Anspruch 5, bei der der Polyalkohol Glycerin aufweist.

7. Wundabdeckung nach Anspruch 6, bei der das Glycerin in einer Menge von etwa 25 bis 30 Gew.-% vorliegt.

8. Wundabdeckung nach einem der Ansprüche 5, 6 oder 7, bei der das Salz Natriumchlorid ist.

9. Wundabdeckung nach einem der vorangegangenen Ansprüche, bei der das Hydrogel (12, 24) eine klare Hydrogelschicht aufweist.

10. Wundabdeckung nach einem der vorangegangenen Ansprüche, die einen gedruckten Wundenmaßstab (20, 32) auf der Rückenbeschichtung (14, 28) aufweist und das Hydrogelmaterial (12, 24) bedeckt.

11. Verfahren zur Herstellung einer Wundabdeckung (10) mit einem flexiblen Film (14), der mit einem drucksensitiven Klebemittel (16), welches auf der Haut eines Patienten haften kann, beschichtet ist, einem absorbierenden Material (12) und einer Abziehlage (18), die sich über das drucksensitive Klebemittel und das absorbierende Material erstreckt, **gekennzeichnet** durch die Schritte, in denen die nichthaftend beschichtete Seite des haftend beschichteten, flexiblen Films (14) auf einer Vakuumplatte (36) mit einer darin geformten Vertiefung (38) angebracht wird, an der Vakuumplatte ein Vakuum gezogen wird, um den mit Klebemittel beschichteten, flexiblen Film (14) in die Vertiefung zu ziehen, ein absorbierendes Material in Form eines Hydrogels (12) in die Vertiefung, die in dem mit Klebemittel beschichteten, flexiblen Film entsteht, gegeben und die Abziehlage (18) auf dem mit Klebemittel beschichteten, flexiblen Film und der offenen Hydrogeloberfläche angebracht wird.

12. Verfahren nach Anspruch 11, bei dem das Hydrogelmaterial (12) etwa 20 bis 35 Gew.-% Polyalkohol, ausgewählt aus der Gruppe, bestehend aus Polypropylenglycol, Polyethylenglycol und Glycerin, etwa 8 bis 12 Gew.-% durch Isophorondiisocyanat terminiertes Vorpolymerisat mit einem Gehalt von etwa 3 % NCO, etwa 5 bis 7 Gew.-% auf Polyethylenoxid basierendes Diamin, bis etwa 1 Gew.-% Salz und Wasser umfaßt.

13. Verfahren nach Anspruch 12, bei dem das Salz Natriumchlorid ist.

14. Verfahren nach einem der Ansprüche 11, 12 oder 13, bei dem das Hydrogel (12) etwa 25 bis 30 Gew.-% Glycerin enthält.

15. Verfahren nach einem der vorangegangenen Ansprüche 11 bis 14, das den Schritt umfaßt, in dem auf den flexiblen Film (14) ein Wundenmaßstab (20) gedruckt wird, der das Hydrogelmaterial (12) bedeckt.

## Revendications

1. Pansement de blessure (10, 22) comprenant une couche de renforcement flexible (14, 28) comportant une couche adhésive sensible à la pression (16, 29), un matériau absorbant (12, 24) sur le côté blessure de la couche de renforcement, et une garniture détachable (18, 30) s'étendant au dessus de la couche adhésive sensible à la pression exposée (16, 29) et du matériau absorbant, ladite garniture détachable pouvant être détachée de manière sélective de sorte qu'elle peut être retirée du pansement de blessure intacte pour laisser exposées des parties de l'adhésif sensible à la pression (16, 29) et du matériau absorbant (12, 24), caractérisé par une dépression sur le côté blessure du pansement et un matériau hydrogel (12, 24) constituant le matériau absorbant contenu dans la dépression.

2. Pansement de blessure (22) selon la revendication 1, dans lequel la dépression est formée par une couche de barrage (26) définissant un périmètre qui recouvre la couche de renforcement (28).

3. Pansement de blessure selon la revendication 1 ou 2, dans lequel la couche de renforcement (14, 28) comprend un film de polyuréthanne.

4. Pansement de blessure selon la revendication 1, 2 ou 3, dans lequel l'adhésif sensible à la pression (16, 29) comprend un adhésif de copolymère acrylique.

5. Pansement de blessure selon la revendication 1, 2, 3 ou 4, dans lequel le matériau hydrogel (12, 24) comprend d'environ 20% à environ 35% en poids d'un alcool polyhydrique choisi dans le groupe constitué du polypropylène glycol, polyéthylène glycol et glycérine, d'environ 8% à environ 12% en poids d'un prépolymère terminé à l'isophoronediisocyanate avec environ 3% de teneur en NCO, d'environ 5% à environ 7% en poids d'une diamine à base d'oxide de polyéthylène, jusqu'à environ 1% en poids dun sel, et d'eau.

6. Pansement de blessure selon la revendication 5, dans lequel l'alcool polyhydrique comprend de la glycérine.

7. Pansement de blessure selon la revendication 6, dans lequel la glycérine est présente dans une quantité allant d'environ 25% à environ 30% en poids.

8. Pansement de blessure selon la revendication 5, 6, ou 7, dans lequel le sel est du chlorure de sodium.

9. Pansement de blessure selon l'une quelconque des revendications précédentes, dans lequel l'hydrogel (12, 24) comprend une couche d'hydrogel transparente.

10. Pansement de blessure selon l'une quelconque des revendications précédentes, comprenant un calibreur de blessure imprimé (20, 32) sur la couche de renforcement (14, 28) et recouvrant le matériau hydrogel (12, 24)

11. Procédé de fabrication d'un pansement de blessure (10) ayant un film flexible (14) revêtu d'un adhésif sensible à la pression (16) capable d'adhérer à la peau d'un patient, un matériau absorbant (12) et une garniture détachable (18) s'étendant au-dessus de la couche sensible à la pression et du matériau absorbant, caractérisé par les étapes d'introduire le côté revêtu non adhésif du film flexible revêtu d'adhésif (14) dans un cadre presseur à vide (36) ayant une cavité (38), de créer un vide dans le cadre presseur à vide pour attirer le film flexible revêtu d'adhésif (14) dans la cavité, de distribuer un matériau absorbant sous la forme d'un hydrogel (12) dans la cavité formée dans le film flexible revêtu d'adhésif, et de fixer la garniture détachable (18) au dessus de la surface du film flexible revêtu d'adhésif exposé à l'hydrogel.

12. Procédé selon la revendication 11, dans lequel le matériau hydrogel (12) comprend d'environ 20% à environ 35% en poids d'un alcool polyhydrique choisi dans le groupe constitué du polypropylène glycol, polyéthylène glycol et glycérine, d'environ 8% à environ 12% en poids d'un prépolymère terminé l'isophoronediisocyanate avec environ 3% de teneur en NCO, d'environ 5% à environ 7% en poids d'une diamine à base d'oxide de polyéthylène, jusqu'à environ 1% en poids d'un sel, et d'eau.

13. Procédé selon la revendication 12, dans lequel le sel est du chlorure de sodium.

14. Procédé selon la revendication 11, 12 ou 13, dans lequel l'hydrogel (12) comprend d'environ 25% à environ 30% en poids de glycérine.

15. Procédé selon l'une quelconque des revendications 11 à 14, comprenant l'étape d'imprimer un calibreur de blessure (20) sur le film flexible (14) et de recouvrir le matériau hydrogel (12).
